(19) Europäisches Patentamt
European Patent Office
Office européen des brevets

(11) **EP 1 098 615 B1**

(12) **EUROPEAN PATENT SPECIFICATION**

(45) Date of publication and mention
of the grant of the patent:
**09.04.2003 Bulletin 2003/15**

(51) Int Cl.⁷: **A61F 13/15**

(21) Application number: **99933425.3**

(86) International application number:
**PCT/SE99/01201**

(22) Date of filing: **01.07.1999**

(87) International publication number:
**WO 00/001334 (13.01.2000 Gazette 2000/02)**

(54) **LIQUID-PERMEABLE SURFACE MATERIAL FOR ABSORBENT DISPOSABLE PRODUCTS, METHOD FOR THE MANUFACTURE OF THIS, AND AN ABSORBENT DISPOSABLE PRODUCT WITH SUCH A SURFACE MATERIAL**

FLÜSSIGKEITSDURCHLÄSSIGES OBERFLÄCHENMATERIAL FÜR SAUGFÄHIGE WEGWERFARTIKEL, VERFAHREN ZU SEINER HERSTELLUNG UND DIESES OBERFLÄCHENMATERIAL ENTHALTENDES SAUGFÄHIGES PRODUKT

MATIERE DE SURFACE PERMEABLE AU LIQUIDE DESTINEE A DES PRODUITS ABSORBANTS JETABLES, SON PROCEDE DE PRODUCTION ET PRODUIT ABSORBANT JETABLE DOTE D'UNE TELLE MATIERE DE SURFACE

(84) Designated Contracting States:
**AT BE CH CY DE DK ES FI FR GB GR IE IT LI LU MC NL PT SE**

(30) Priority: **02.07.1998 SE 9802365**

(43) Date of publication of application:
**16.05.2001 Bulletin 2001/20**

(73) Proprietor: **SCA Hygiene Products AB**
**405 03 Göteborg (SE)**

(72) Inventor: **KRISTIANSEN, Björn**
**S-434 94 Vallda (SE)**

(74) Representative: **Romare, Laila Anette**
**Albihns Göteborg AB**
**Box 142**
**401 22 Göteborg (SE)**

(56) References cited:
**EP-A1- 0 604 731          EP-A2- 0 903 136**
**GB-A- 2 014 508           US-A- 5 366 782**

## Description

This invention relates to a liquid-permeable surface material for absorbent disposable products, such as nappies, incontinence pads, sanitary towels, panty liners or the like. The invention also relates to a method for the manufacture of such a surface material and an absorbent disposable product which utilizes such a surface material.

CURRENT TECHNOLOGY:

Various types of liquid-permeable surface materials are already known for use on the face of absorbent disposable products which is designed to be next to the crutch area of the wearer when in use.

Common liquid-permeable surface materials are various textile materials and nonwoven fabric materials, perforated plastic film and mesh materials (see e.g. GB 2014508 A).

The liquid-permeable surface material on an absorbent disposable product has several functions.

An important function is to allow excreted bodily fluids to pass rapidly through to an underlying absorbent core. In certain cases there is therefore a dispersing layer or spacing layer between the liquid-permeable surface material and the absorbent core.

Another important function of the liquid-permeable material is to feel dry and comfortable against the wearer's skin. For this reason many liquid-permeable surface materials are currently hydrophobic.

A third important function of the liquid-permeable surface material is to provide an aesthetically-pleasing surface even after use, that is to conceal excreted bodily fluids which have been absorbed by the underlying absorbent core.

Although a very thin hydrophobic material, for example a plastic mesh, can fulfil the requirements concerning allowing liquid to pass through rapidly and providing a dry and comfortable surface for contact with the skin, such a material can scarcely fulfil the function of concealing excreted bodily fluids.

Current liquid-permeable surface materials for absorbent disposable products are therefore generally quite dense, which sometimes reduces the rate at which liquid is able to pass through to a level which is insufficient for certain applications, for example incontinence pads where there can be momentarily high flows of urine.

For this reason, among others, absorbent disposable products have largely started to be provided with various types of projecting barrier devices and the like on the surface of the absorbent disposable products in order to ensure that large quantities of bodily fluids can be trapped and do not soil the wearer's clothes.

Another proposed way of improving how liquid passage through relatively dense liquid-permeable surface materials is to provide a porous fibre-wadding layer under the liquid-permeable surface material in order to create volume. A disadvantage of introducing a porous wadding layer is of course that it involves additional cost.

With the aim of achieving a more effective transportation of liquid through the liquid-permeable surface material it has also been proposed that a pore size gradient or a hydrophilic/hydrophobic gradient should be created through the thickness of the porous wadding under the liquid-permeable material. Such solutions can, however, be difficult to put into practice.

DESCRIPTION OF THE INVENTION:

A first aim of this invention is thus to provide a liquid-permeable surface material for use on the face of an absorbent disposable product which is next to a wearer in use, which surface material can be obtained by means of a simple manufacturing process and which avoids the abovementioned problems concerning insufficient liquid being allowed to pass through and at the same time is able to conceal bodily fluids absorbed in an underlying absorbent core.

This aim is achieved according to the following Patent Claim 1, by means of the liquid-permeable surface material comprising a material structure in micro-scale with many pores. Each of the pores has, in the plane of the surface material, a first pore cross section dimension, a second pore cross section dimension, a pore cross section area and a pore circumference, where the second pore cross section dimension is essentially at right angles to the first pore cross section dimension. According to the invention, the stretched and thereby permanently deformed and in this condition fixed liquid-permeable surface material has a first pore cross section dimension which is considerably larger than the second pore cross section dimension as a result of the said stretching process, thereby obtaining a low pressure drop when fluid flows through.

By the term "material structure in micro-scale with many pores" is understood here the type of material structure with pores which is formed from the fibres which normally make up, for example, a nonwoven fabric material. The term does not cover pore structures in "macro-scale" which are visible to the naked eye which have been formed by means of perforating, bonded patterns, embossing and the like. An indication of the size of the pores which are meant by the term "micro-scale" is pores with pore cross section dimensions (external pore diameter) in the range 1 μm - 150 μm.

A second aim of this invention is to provide a liquid-permeable surface material which both avoids the abovementioned problem concerning allowing insufficient liquid to pass through and at the same time has an optimal ability to conceal absorbed bodily fluids.

This second aim is achieved according to the following Patent Claim 2, in that as a result of the stretching process, the pore cross section area of the liquid-permeable surface material has been reduced and the pore circumference has been retained essentially unchanged in comparison to before the stretching process.

A third aim of this invention is to provide a method of manufacturing a liquid-permeable surface material according to the invention.

This third aim of this invention is achieved according to the following Patent Claim 6, in that the method comprises providing a liquid-permeable material web with a material structure in micro-scale with many pores. Each of the many pores has thereby a first pore cross section dimension, a second pore cross section dimension, a pore cross section area and a pore circumference, where the second pore cross section dimension is essentially at right angles to the first pore cross section dimension, and the material web has a longitudinal direction and a transverse direction. According to the invention the material web is subjected to a stretching process under tension by at least 20% in either the said longitudinal direction or said transverse direction so that the first pore cross section dimension becomes considerably larger than the second pore cross section dimension, after which the material structure obtained is fixed in order to provide a liquid-permeable surface material for absorbent disposable products with a reduced pressure drop when liquid flows through.

A fourth aim of this invention is to provide an absorbent disposable product which utilizes a liquid-permeable surface material according to the invention.

This fourth aim of this invention is achieved according to the following Patent Claim 11, in that the absorbent disposable product has a longitudinal direction and a transverse direction and comprises a front part and a back part with a crutch part between them. The disposable product also comprises a front-face material, intended to be in contact with the wearer's skin when in use, a back-face material intended to be in contact with the wearer's clothes when in use, and an absorbent core lying between the front-face material and the back-face material. The front-face material comprises, at least in connection with the crutch part, a liquid-permeable surface material which comprises a material structure in micro-scale with many pores. Each of the many pores has, in the plane of the surface material, a first pore cross section dimension, a second pore cross section dimension, a pore cross section area and a pore circumference, where the second pore cross section dimension is essentially at right angles to the first pore cross section dimension. According to the invention the liquid-permeable surface material is subjected to a stretching process before use in the absorbent disposable product in order to obtain a low pressure drop when liquid flows through, whereby the first pore cross section dimension becomes considerably larger than the second pore cross section dimension as a result of the said stretching process.

Further aims of the invention will become obvious from this description while the special characteristics which enable the further aims to be achieved are defined in the attached subordinate patent claims.

BRIEF DESCRIPTION OF THE FIGURES:

In the following the invention will be described in greater detail with reference to the attached figures, where

Figure 1   shows diagrammatically and greatly simplified in micro-scale a conventional liquid-permeable material which can also comprise the raw material for the manufacture of a liquid-permeable surface material according to the invention,

Figure 2   shows diagrammatically and greatly simplified in micro-scale a liquid-permeable surface material according to the invention, originating from the raw material in Figure 1 which has been subjected to a method according to the invention, and

Figure 3   shows a perspective view of an absorbent disposable product which utilizes the liquid-permeable surface material according to the invention on the face of the product which is intended to be facing the crutch area of a wearer in use.

DETAILED DESCRIPTION OF PREFERRED EMBODIMENTS

The pressure drop ($\Delta P_d$) for a flow through an absorbent structure can generally be calculated using the formula

$$(1) \qquad \Delta P_d = 1/2 \; \xi \cdot \omega^2$$

where $\xi$ is a coefficient of resistance and $\omega$ is the rate of flow of the liquid passing through.

In the case of thin layers of material, for example liquid-permeable surface materials for absorbent disposable products, the pressure drop ($\Delta P_d$) through the surface material can be approximated by the formula

$$(2) \quad \Delta P_d \approx 2\, d_h$$

where $d_h$ refers to the hydraulic diameter.

The hydraulic diameter $d_h$ can be calculated using the formula

$$(3) \quad d_h = 4A/S$$

where A is the average cross section area and S is the average circumference of the capillaries/pores in the layer of the material.

Thus the pressure drop through a liquid-permeable thin material should be able to be approximated using the formula

$$(4) \quad \Delta p_d \approx 8A\,/\,S$$

which can be derived from the above formulas (2) and (3).

In practice this means that a liquid-permeable surface material with pores/capillaries with a large cross section area in relation to their circumference will give a high pressure drop, while a liquid-permeable surface material with pores/capillaries with a small cross section area in relation to their circumference will give a low pressure drop.

This fact is illustrated by the two examples below.

Example 1

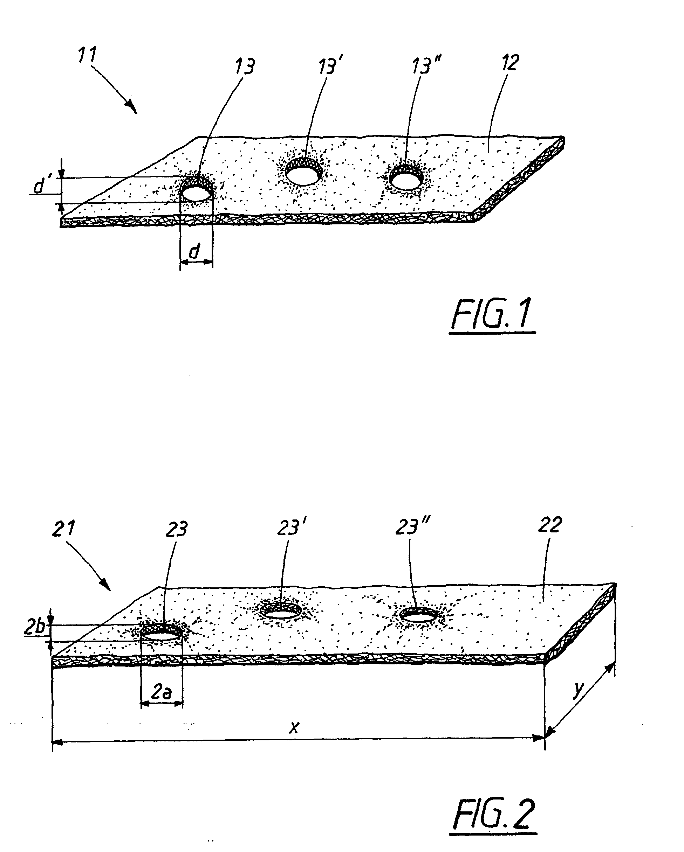

Figure 1 shows diagrammatically and greatly simplified in micro-scale a conventional liquid-permeable surface material 11, which can also comprise the raw material for a liquid-permeable surface material according to the invention.

The conventional surface material 11 comprises a material structure 12 containing many through pores 13, 13', 13", which in the ideal case shown diagrammatically in Figure 1 have a circular cross section in the plane of the surface material.

The cross section area $A_1$, in the plane of the surface material, of the pores 13, 13', 13" in the material structure 12 can be calculated using the formula:

$$(5) \quad A_1 = \pi \cdot d^2/4$$

and the circumference S using the formula:

$$(6) \quad S = \pi \cdot d$$

If the circumference of the pores 13, 13', 13" is for example on average 20 $\mu$m, the average diameter d in the plane of the surface material is obtained from the formula (6) d = S / $\pi$ = 20 / $\pi \approx$ 6.4 $\mu$m, and the average cross section area will thus be according to formula (5)

$$A_1 = \pi \cdot d^2 \,/\, 4 = \pi \cdot 6.4^2 \,/\, 4 \approx 32.2\ \mu m^2$$

According to the abovementioned formula (4) the approximate pressure drop across the conventional surface material 11 in the example will be $\Delta p_d$ = 8A$_1$ / S $\approx$ 8 · 32.2 / 20 $\approx$ 12.9 for a flow of liquid through the surface material 11.

Example 2

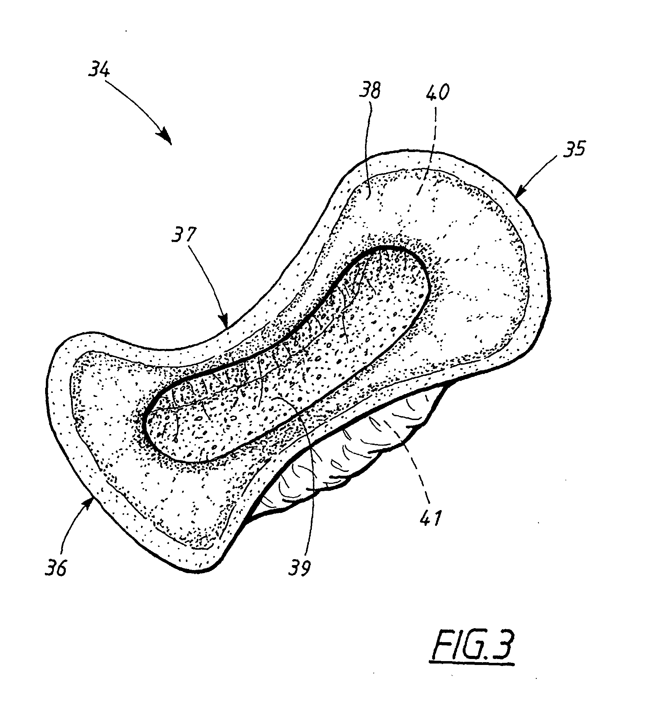

Figure 2 shows diagrammatically and greatly simplified in micro-scale a liquid-permeable surface material 21 according to the invention which has been subjected to a method according to the invention. In this example, the conventional surface material shown in Figure 1 has been permanently deformed by a stretching process using the method according to the invention, so that the length X of the surface material has been increased by approximately 30%, while the width Y has been reduced to a corresponding extent.

The liquid-permeable surface material 21 according to the invention shown in Figure 2 comprises a material structure 22 containing many through pores 23, 23', 23", which in the ideal case shown diagrammatically in Figure 2 have been given a lengthened, essentially elliptical cross section in the plane of the surface material 21 by means of the method according to the invention.

The circumference of each of the pores 23, 23', 23" shown in Figure 2 can be approximated using the formula:

$$(7) \quad S \approx 2\pi \cdot ( 1/2 \cdot (a^2+b^2) )^{0.5}$$

while the cross section area in the plane of the surface material for each of the pores 23, 23', 23 " can be calculated by using the formula:

$$(8) \quad A_2 = \pi \cdot a \cdot b$$

The liquid-permeable surface material 21 according to the invention has as mentioned above been stretched, so that the length X of the material has been increased by approximately 30%, while the width of the material Y has been reduced to a corresponding extent.

Based on this fact it can be assumed that the cross section of the pores 23, 23', 23" in the plane of the surface material has been deformed to approximately the same extent. Based on the average pore diameter 6.4 in the conventional surface material 11 discussed in Example 2 this would mean that $2a \approx 1.3 \cdot 6.4 \approx 8.3$ for a pore 23 in the surface material 21 according to the invention shown in Figure 2. As it can be assumed that b has been reduced to a corresponding extent as a has been increased, it can be estimated that $2b \approx 4.5$ and thus that $a \approx 4.15$ and $b \approx 2.25$.

Based on these approximate values of a and b for the elliptical cross section of the pores in the liquid-permeable surface material according to the invention in Figure 2, the cross section in the plane of the surface material according to formula (8) is calculated as $A_2 = \pi \cdot 4.15 \cdot 2.25 \approx 29.3$.

If it is assumed that the pore circumference S remains unchanged, the liquid-permeable surface material according to the invention shown in Figure 2 has thus according to formula (4) the estimated pressure drop $\Delta p_d \approx 8A_2$ / $S \approx 8 \cdot 29.3 / 20 \approx 11.7$, which is lower than the value of 12.9 obtained in Example 1.

This surprising result, that is to say the fact that the pressure drop across a liquid-permeable surface material can be reduced by giving the pores of the surface material a more elongated shape, forms the basis for this invention.

This relationship can also be expressed as the fact that each pore in a surface material in the plane of the surface material has a first and a second pore cross section dimension which are essentially at right angles to each other, and that each pore has in addition a pore cross section area and a pore circumference in the plane of the surface material. In the surface material according to the invention, compared to conventional surface material, the first pore cross section dimension has become considerably larger than the second pore cross section dimension.

Thanks to the abovementioned relationship the surface material according to the invention provides a lower pressure drop for the flow of liquid than conventional surface materials and in addition provides aesthetic benefits, as the pore cross section area is less than in conventional surface materials so that, for example, bodily fluids which have been absorbed in an underlying absorbent core in an absorbent disposable product can be better concealed.

In particularly preferred embodiments of the surface material according to the invention it is also the case that the pores in the surface material have been given a smaller cross section area in the plane of the surface material with an essentially unchanged circumference. This makes it possible, as described above, for the surface material to provide an optimal concealing effect concerning preventing bodily fluids, which have been absorbed in an absorbent core under the surface material, being visible to the wearer.

With the aim of illustrating in a clear way the above effect which forms the basis for this invention, the following TABLE 1 shows pressure drop values estimated in accordance with formula (4) for a number of different liquid-permeable surface materials according to the invention (INVENTION) and also for three reference materials (REFERENCE) in the form of conventional liquid-permeable surface materials. In addition TABLE 1 shows the estimated pressure drop values for surface materials according to the invention (INVENTION II) which approach the limit values stated in the attached Patent Claim 3. The pressure drop values in TABLE 1 have been calculated using the same method of calculation as in Examples 1 and 2 above.

TABLE 1

| Surface material | Pore cross section | Circumference | Deformation | Pressure drop |
|---|---|---|---|---|
| *REFERENCE* | *Circular* | *20* | *0%* | *12.9* |
| INVENTION | Elliptical | 20 | 20% | 12.3 |
| INVENTION | Elliptical | 20 | 30% | 11.7 |
| INVENTION | Elliptical | 20 | 40% | 10.8 |
| INVENTION | Elliptical | 20 | 50% | 9.6 |
| INVENTION | Elliptical | 20 | 60% | 8.2 |
| INVENTION | Elliptical | 20 | 70% | 6.6 |
| INVENTION | Elliptical | 20 | 80% | 4.6 |
| INVENTION | Elliptical | 20 | 90% | 2.4 |
| INVENTION II | elliptical a/b = 1.8-1.9 | **22** | 30% | **12.7** |
| *REFERENCE* | *Circular* | *25* | *0%* | *16.1* |
| INVENTION | Elliptical | 25 | 20% | 15.3 |
| INVENTION | Elliptical | 25 | 50% | 11.9 |
| INVENTION | Elliptical | 25 | 90% | 3.0 |
| INVENTION II | elliptical a/b= 1.8-1.9 | **27.5** | 30% | **15.9** |
| *REFERENCE* | *Circular* | *30* | *0%* | *19.1* |
| INVENTION | Elliptical | 30 | 50% | 14.3 |
| INVENTION II | elliptical a/b = 1.8-1.9 | **33** | 30% | **19.1** |

The above TABLE 1 shows that the estimated pressure drop is lower for the liquid-permeable surface material according to the invention than for the three conventional surface materials.

When optimizing the pressure drop reduction and improving the aesthetic/concealing characteristics of the surface material according to the invention, the degree of deformation must be adapted in each individual case, for example dependent upon the raw material which is to be subjected to the method according to the invention.

The liquid-permeable surface material according to the invention can in principle be manufactured according to the method according to the invention from any suitable conventional surface material for absorbent products, for example a spun-bond material or a drawn thermally-bound nonwoven material. Perforated plastic films or other suitable raw materials can also be used for the manufacture of the surface material according to the invention. In addition, both hydrophilic and hydrophobic materials can be considered as surface materials for the manufacture of the liquid-permeable surface material according to the invention.

In the case where the raw material, which is used for the surface material according to the invention, is very hydrophobic, there are however limitations on how great a deformation the raw material should be subjected to in practice. The reason for this is that the flow of liquid through a hydrophobic material, unlike a hydrophilic material, is also dependent upon what is called the bridging pressure, which depends among other things on the pore dimensions, the hydrophobic properties and the thickness of the hydrophobic material concerned. In such a case there is a minimum value for the second, smaller pore cross section dimension of the pores in the surface material according to the invention. If the second pore cross section dimension is less than the minimum value, a hydrophobic surface material will become virtually impermeable to liquid, for which reason the abovementioned minimum value must be taken into account when selecting the amount of deformation which is to be applied when stretching a hydrophobic raw material according to the method according to the invention.

It can be difficult to avoid a certain, in many cases unwanted, increase in the pore circumference occurring during the method according to the invention. As shown in TABLE 1 (INVENTION II) the method according to the invention should preferably be implemented in such a way that the ratio between the first pore cross section dimension and the second pore cross section dimension is at least 1.8 in the finished surface material according to the invention, and that the pore circumference is at most 110% of the pore circumference prior to the stretching process. This guarantees that the method according to the invention provides a reduced pressure drop across the liquid-permeable surface material and at the same time gives the material an adequate ability to conceal bodily fluids absorbed in an underlying absorbent core.

In addition, the maximum practicably useable amount of deformation depends on the strength and elasticity of the raw material used and must therefore be adapted to suit in each individual case.

The method according to the invention is described below with reference to the attached Figures 1 and 2, when applicable.

The method according to the invention can be implemented using arrangements comprising components known per se, for example a frame with suitably arranged unrolling and rolling-up devices, carrier rollers, pinch rollers and the like, whereby an adequate web tension can be achieved in a material web of liquid-permeable raw material in order to stretch and permanently deform the material web and fix it in this condition in order thereby to obtain a liquid-permeable surface material according to the invention.

The method according to the invention comprises in a first preferred embodiment providing a liquid-permeable material web 11 with a material structure 12 in micro-scale with many pores 13, 13', 13". The liquid-permeable material web 11, which constitutes the raw material for the method according to the invention can, as previously mentioned, in principle consist of any liquid-permeable material already known per se and consists preferably of a liquid-permeable nonwoven fabric material.

Each of the pores 13 of the many pores 13, 13', 13" in the material web 11 has a first pore cross section dimension d, a second pore cross section dimension d', a pore cross section area and a pore circumference, where the second pore cross section dimension d' is essentially at right angles to the first pore cross section dimension d and the material web 11 which is to be subjected to the method according to the invention has a longitudinal direction and a transverse direction.

In the method according to the invention, the material web 11 is stretched under tension by at least 20% in either the said longitudinal direction or the said transverse direction.

This leads in the described embodiment to the pore cross section area becoming smaller as the first pore cross section dimension 2a becomes considerably larger than the second pore cross section dimension 2b (Figure 2), while the pore circumference of the pores in the material web remains essentially unchanged. However, there can also be embodiments of the invention where the pore circumference is allowed to increase to some extent, even though this can of course make the finished surface material less effective at concealing bodily fluids.

The material structure 22 obtained in the material web is then fixed in a suitable way in order to provide a liquid-permeable surface material 21 for absorbent disposable products with a reduced pressure drop for liquids flowing through.

According to a preferred embodiment of the invention, the raw material web 11 comprises thermoplastic material with a softening temperature. The material web 11 is heated up before or during the stretching process to a temperature which is higher than the softening temperature, after which the material structure 22 obtained after the stretching process is fixed by being cooled to a temperature which is lower than the softening temperature while retaining the web tension.

In a further preferred embodiment of the method the material web comprises cellulose-based fibres. In this embodiment the material web 11 is moistened before or during the stretching process by the application of water, after which the material structure 22 obtained after the stretching process is fixed by evaporating off the water while retaining the web tension so that fixing paper bonds are formed in the material structure 22.

A further preferred embodiment of the method comprises the use of a binding agent which is applied to the material web before, during or after the stretching process, after which the material structure 22 is fixed after the stretching process by cross-linking the binding agent.

In the following a first preferred embodiment of an absorbent disposable product according to the invention is described with reference to the attached Figure 3.

The disposable product 34 according to the first embodiment has a longitudinal direction and a transverse direction and comprises a front part 35 and a back part 36 with a crutch part 37 between them.

The disposable product 34 also comprises in the described embodiment a front-face material 38, intended to be in contact with the wearer's skin when in use. The front-face material 38 consists in the described embodiment of a liquid-repelling nonwoven fabric material with an opening covered by a liquid-permeable surface material 39 according to the invention.

The disposable product 34 also comprises a back-face material 40 intended to be in contact with the wearer's clothes when in use. The back-face material 40 can be any suitable material according to any known technology, but is preferably a liquid-impermeable plastic film and in the described embodiment a polyethylene film.

The disposable product 34 also comprises an absorbent core 41 lying between the front-face material 38 and back-face material 40. The absorbent core 41 can be of any conventional type, and comprises in the described embodiment a mixture of cellulose wadding and a superabsorbent material of the starch-based type.

According to the described embodiment a piece of liquid-permeable surface material 39 according to the invention is attached to the front-face material 38 on the side which is facing the absorbent core 41 whereby the liquid-permeable surface material 39 forms an area where liquid can pass through in the crutch area 37 of the disposable product. In the described embodiment the liquid-permeable surface material 39 only covers a part of the side of the front-face material 38 which is facing the absorbent core 41.

However, there could be other embodiments of the invention where the whole of the front-face material 38 consists of liquid-permeable surface material according to the invention, or where the edges of the opening are provided with elasticized barriers (not shown in Figure 3) which project from the plane of the front-face material 38 around the opening covered by the liquid-permeable surface material 39.

The front-face material 38 in the absorbent disposable product 34 according to the invention comprises, at least in connection with the crutch part 37, a liquid-permeable surface material 39 according to the invention of the type described above.

The absorbent disposable product 34 is characterized in the described embodiment of the invention by the liquid-permeable surface material 39 being subjected to a stretching process before being applied onto the absorbent disposable product 34 in order to provide a low pressure drop when liquid passes through. The stretching process results in the first pore cross section dimension becoming considerably larger than the second pore cross section dimension.

In a particularly preferred embodiment of the absorbent disposable product 34 according to the invention the pore cross section area has been reduced and the pore circumference has remained essentially unchanged, in comparison to before the stretching process.

In another preferred embodiment of the absorbent disposable product 34 according to the invention the pressure drop through the liquid-permeable surface material 39 is estimated using the formula eight times the pore cross section area divided by the pore circumference. According to this embodiment the first pore cross section dimension in the liquid-permeable surface material 39 on the absorbent disposable product 34 is at least 1.8 times larger than the second pore cross section dimension, while the pore circumference is at most 110% of the pore circumference before the stretching process to which the liquid-permeable surface material 39 was subjected before being applied onto the absorbent disposable product 34. This embodiment provides an absorbent disposable product 34 with an improved ability to take in bodily fluids which at the same time can effectively conceal the bodily fluids which have been absorbed by the absorbent core 41 inside the product when the absorbent disposable product 34 is in use.

In another preferred embodiment of the absorbent disposable product 34 according to the invention, before being applied onto the absorbent disposable product 34, the liquid-permeable surface material 39 has a longitudinal direction and a transverse direction and comprises thermoplastic material with a softening temperature. According to this embodiment before being applied onto the absorbent disposable product 34 the liquid-permeable surface material 39 is heated up to a temperature higher than the softening temperature and is then stretched under tension by at least 20% in either the longitudinal or transverse direction. In this embodiment the liquid-permeable surface material 39 is then cooled to a temperature lower than the softening temperature while retaining the tension.

In a further preferred embodiment of the absorbent disposable product according to the invention, before the liquid-permeable surface material 39 is applied onto the absorbent disposable product 34, the material structure of the liquid-permeable surface material 39 is fixed in a stretched condition by dampening with water and drying, or by the application of a binding agent and the setting of the binding agent.

The invention is not restricted to the embodiments described above and shown in the Figures, but a number of further variants and modifications are possible within the framework of the following patent claims.

For example, the absorbent disposable product according to the invention can of course be provided with a suitable attachment device according to known technology in order to enable the product to be fastened together into a panty shape in use and to be undone again.

Any elastic device used on the front-face material of an absorbent disposable product according to the invention can of course, if so required, also work together with other elastic devices on an absorbent disposable product in which a liquid-permeable surface material according to the invention constitutes at least part of the front-face material.

**Claims**

1. A liquid-permeable surface material for absorbent disposable products, said surface material (21) comprising a material structure (22) in micro-scale with many pores (23, 23', 23"), with pore cross section dimensions in the range 1 μm - 150 μm, each (23) of the said pores with, in the plane of the surface material (21), a first pore cross section dimension (2a), a second pore cross section dimension (2b), a pore cross section area and a pore circumference, where said second pore cross section dimension (2b) is essentially at right angles to said first pore cross section dimension (2a) **characterized in that** in either longitudinal or transverse direction stretched and thereby permanently deformed and in this condition fixed liquid-permeable surface material (21) has a first pore cross section dimension (2a) which is considerably larger than the second pore cross section dimension (2b), thereby obtaining a low pressure drop when liquid flows through.

2. A liquid-permeable surface material according to Claim 1, **characterized in that** as a result of the stretching process the pore cross section area has been reduced and the pore circumference has remained essentially unchanged compared to before said stretching process.

3. A liquid-permeable surface material according to Claim 1 or 2, in which the pressure drop is estimated according to the formula eight times the pore cross section area divided by the pore circumference **characterized in that** the first pore cross section dimension (2a) upon application of the liquid-permeable surface material (21) in an absorbent disposable product is at least 1.8 times larger than the second pore cross section dimension (2b), and that the pore circumference is at most 110% of the pore circumference before the stretching process.

4. A liquid-permeable surface material according to anyone of Claims 1 - 3, the said surface material (21) having a longitudinal direction (X) and a transverse direction (Y) and comprising thermoplastic material with a softening temperature, **characterized in that** during said stretching process the liquid-permeable surface material (21) has been heated up to a temperature higher than said softening temperature and has been stretched under tension by at least 20% in either said longitudinal direction (X) or said transverse direction (Y), and that in connection with said stretching process the liquid-permeable material (21) has been cooled down to a temperature lower than aid softening temperature while retaining said tension.

5. A liquid-permeable surface material according to any of Claims 1 - 3, **characterized in that** in connection with said stretching process the material structure (22) of the liquid-permeable surface material (21) has been fixed in a stretched condition by dampening with water and drying, or by the application of a binding agent and setting the binding agent.

6. A method for manufacturing a liquid-permeable surface material for absorbent disposable products, said method comprising providing a liquid-permeable material web (11) with a material structure (12) in micro-scale with many pores (13, 13', 13"), each (13) of said many pores having a first pore cross section dimension (d), a second pore cross section dimension (d'), a pore cross section area and a pore circumference, where said second pore cross section dimension (d') is essentially at right angles to said first pore cross section dimension (d) and the material web (11) has a longitudinal direction and a transverse direction, **characterized in that** the material web (11) is permanently deformed by subjecting it to a stretching process under tension by at least 20% in either said longitudinal direction or said transverse direction so that the first pore cross section dimension (2a) becomes considerably larger than the second pore cross section dimension (2b) and that thereafter the material structure (2b) of the material web obtained is fixed in order to provide a liquid-permeable surface material (21) for absorbent disposable products with a reduced pressure drop when liquid passes through.

7. A method according to Claim 6, **characterized in that** the pore cross section area is reduced and the pore circumference remains essentially unchanged after said stretching and fixing processes.

8. A method according to Claim 6 or 7, in which the material web (11) comprises thermoplastic material with a softening temperature, **characterized in that** before or during said stretching process the material web is heated up to a temperature higher than said softening temperature, and that after the stretching process the material structure (22) is fixed by cooling to a temperature lower than said softening temperature while maintaining said tension.

9. A method according to Claim 6 or 7, in which the material web (11) comprises cellulose-based fibres, **characterized in that** before or during said stretching process the material web is moistened by the application of water, and that after the stretching process the material structure (22) is fixed by evaporating off said water by drying while maintaining said tension so that fixing paper bonds are formed in said material structure (22).

10. A method according to Claim 6 or 7, comprising the use of a binding agent, **characterized in that** said binding agent is applied to the material web before, during or after said stretching process, and that after the stretching process the material structure (22) is fixed by cross-linking said binding agent.

11. An absorbent disposable product, said disposable product (34) having a longitudinal direction and a transverse direction and comprising a front part (35), a back part (36) with a crutch part (37) between them, the disposable product also comprising a front-face material (38) intended to be in contact with the wearer's skin when in use, and a back-face material (40) intended to be in contact with the wearer's clothes when in use, where the disposable product (34) also comprises an absorbent core (41) lying between the said front-face material (38) and back-face material (40) and in which the front-face material (38) comprises, at least in connection with the crutch part (37), a liquid-permeable surface material (39), said surface material (39) comprising a material structure in micro-scale with many pores with pore cross section dimensions in the range 1 μm - 150 μm, where each of the many pores has in the plane of the surface material (39) a first pore cross section dimension, a second pore cross section dimension, a pore cross section area and a pore circumference, where said second pore cross section dimension is essentially at right angles to said first pore cross section dimension, **characterized in that** the liquid-permeable surface material (21) is fixed in a stretched and permanently deformed state and has a first pore cross section dimension (2a) which is considerably larger than the second pore cross section dimension (2b) in order to obtain a low pressure drop when liquid flows through.

12. An absorbent disposable product according to Claim 11, **characterized in that** the pore cross section area has been reduced and the pore circumference remains essentially unchanged, compared to before said stretching process.

13. An absorbent disposable product according to Claim 11 or 12, in which the pressure drop across the liquid-permeable surface material (39) is estimated according to the formula eight times the pore cross section area divided by the pore circumference, **characterized in that** the first pore cross section dimension in the liquid-permeable surface material (39) of the absorbent disposable product (34) is at least 1.8 times larger than the second pore cross section dimension, and the pore circumference is at most 110% of the pore circumference before the stretching process to which the liquid-permeable surface material (39) was subjected before being applied onto the absorbent disposable product (34).

14. An absorbent disposable product according to any of Claims 11 - 13, in which before being applied onto the absorbent disposable product (34) the liquid-permeable surface material (39) has a longitudinal direction and a transverse direction and comprises thermoplastic material with a softening temperature, **characterized in that** before being applied to the absorbent disposable product (34) the liquid-permeable surface material (39) has been heated up to a temperature higher than said softening temperature and has then been stretched under tension by at least 20% in either said longitudinal direction or said transverse direction, and that the liquid-permeable material (39) has also been cooled to a temperature lower than said softening temperature while retaining said tension.

15. An absorbent disposable product according to any of Claims 11 - 13, **characterized in that** before the liquid-permeable surface material (39) is applied onto the absorbent disposable product (34) the material structure of the liquid-permeable surface material (39) has been fixed in a stretched condition by dampening with water and drying, or by the application of a binding agent and setting the binding agent.

**Patentansprüche**

1. Flüssigkeitsdurchlässiges Oberflächenmaterial für absorbierende Wegwerfprodukte, wobei das Oberflächenmaterial (21) im Mikromaßstab eine Materialstruktur (22) mit zahlreichen Poren (23, 23', 23'') aufweist, mit Porenquerschnittsabmessungen in dem Bereich 1 μm bis 150μm, wobei jede (23) der Poren mit, in der Ebene des Oberflächenmaterials (21), einer ersten Porenquerschnittsabmessung (2a), einer zweiten Porenquerschnittsabmessung (2b), einer Porenquerschnittsfläche und einem Porenumfang versehen ist, wobei die zweite Porenquerschnittsabmessung (2b) im wesentlichen unter einem rechten Winkel zu der ersten Porenquerschnittsabmessung (2a) vorliegt, **dadurch gekennzeichnet, dass** das entweder in der Längs- oder in der Querrichtung gedehnte und hierdurch dauerhaft verformte und in diesem Zustand fixierte flüssigkeitsdurchlässige Oberflächenmaterial (21) eine erste Porenquerschnittsabmessung (2a) aufweist, die erheblich größer ist als die zweite Porenquerschnittsabmessung (2b), wodurch ein geringer Druckabfall erhalten wird, wenn Flüssigkeit hindurch fließt.

2. Flüssigkeitsdurchlässiges Oberflächenmaterial nach Anspruch 1, **dadurch gekennzeichnet, dass** als ein Ergebnis des Dehnvorganges die Porenquerschnittsfläche verringert wurde, und der Porenumfang verglichen mit vor dem Dehnvorgang, im wesentlichen unverändert geblieben ist.

3. Flüssigkeitsdurchlässiges Oberflächenmaterial nach Anspruch 1 oder 2, wobei der Druckabfall gemäß der Formel acht mal der Porenquerschnittsfläche, geteilt durch den Porenumfang eingeschätzt wird, **dadurch gekennzeichnet, dass** die erste Porenquerschnittsabmessung (2a) nach Anbringung des flüssigkeitsdurchlässigen Oberflächenmaterials (21) in einem absorbierenden Wegwerfprodukt wenigstens 1,8 mal größer als die zweite Porenquerschnittsabmessung (2b) ist, und dass der Porenumfang höchstens 110% des Porenumfangs vor dem Dehnvorgang ist.

4. Flüssigkeitsdurchlässiges Oberflächenmaterial nach einem der Ansprüche 1 - 3, wobei das Oberflächenmaterial (21) eine Längsrichtung (X) und eine Querrichtung (Y) aufweist, und thermoplastisches Material mit einer Aufweichtemperatur aufweist, **dadurch gekennzeichnet, dass** während des Dehnvorgangs das flüssigkeitsdurchlässige Oberflächenmaterial (21) bis zu einer Temperatur erwärmt wurde, die höher ist als die Aufweichtemperatur, und unter Spannung um wenigstens 20% entweder in der Längsrichtung (X) oder der Querrichtung (Y) gedehnt wurde, und dass in Verbindung mit dem Dehnvorgang das flüssigkeitsdurchlässige Material (21) zu einer Temperatur abgekühlt wurde, die niedriger ist als die Aufweichtemperatur, während die Spannung Zug gehalten wird.

5. Flüssigkeitsdurchlässiges Oberflächenmaterial nach einem der Ansprüche 1 - 3, **dadurch gekennzeichnet, dass** in Verbindung mit dem Dehnvorgang die Materialstruktur (22) des flüssigkeitsdurchlässigen Oberflächenmaterials (21) in einem gedehnten Zustand fixiert wurde, indem es mit Wasser befeuchtet wird und getrocknet wird, oder durch die Aufbringung eines Bindemittels und Setzen des Bindemittels.

6. Verfahren zur Herstellung eines flüssigkeitsdurchlässigen Oberflächenmaterials für absorbierende Wegwerfprodukte, wobei das Verfahren das Vorsehen einer flüssigkeitsdurchlässigen Materialbahn (11) im Mikromaßstab mit einer Materialstruktur (12) mit zahlreichen Poren (13, 13', 13'') aufweist, wobei jede in (13) der zahlreichen Poren eine erste Porenquerschnittsabmessung (d), eine zweite Porenquerschnittsabmessung (d'), eine Porenquerschnittsfläche und einen Porenumfang aufweist, wobei die zweite Porenquerschnittsabmessung(d') im wesentlichen unter einen rechten Winkel zu der ersten Porenquerschnittsabmessung (d) vorliegt, und die Materialbahn (11) eine Längsrichtung und eine Querrichtung aufweist, **dadurch gekennzeichnet, dass** die Materialbahn (11) dauerhaft verformt wird, in dem sie einem Dehnvorgang unter Spannung um wenigstens entweder der Längsrichtung oder der Querrichtung unterworfen wird, so dass die erste Porenquerschnittsabmessung (2a) erheblich größer wird als die zweite Porenquerschnittsabmessung (2b), und dass nachfolgend die Materialstruktur (2b) der Materialbahn, die erhalten wird, fixiert wird, um ein flüssigkeitsdurchlässiges Oberflächenmaterial (21) für absorbierende Wegwerfprodukte zu schaffen, das einen verringerten Druckabfall aufweist, wenn Flüssigkeit hindurch tritt.

7. Verfahren nach Anspruch 6, **dadurch gekennzeichnet, dass** nach dem Dehn- und Fixiervorgang die Porenquerschnittsfläche verringert wird, und der Porenumfang im wesentlichen unverändert bleibt.

8. Verfahren nach Anspruch 6 oder 7, wobei die Materialbahn (11) thermoplastisches Material mit einer Aufweichtemperatur aufweist, **dadurch gekennzeichnet, dass** vor oder während des Dehnvorgangs die Materialbahn zu einer Temperatur erwärmt wird, die höher ist als die Aufweichtemperatur, und dass nach dem Dehnvorgang die Materialstruktur (22) durch Kühlen auf eine Temperatur geringer als die Aufweichtemperatur fixiert wird, während die Spannung gehalten wird.

9. Verfahren nach Anspruch 6 oder 7, wobei die Materialbahn(11) Fasern auf der Basis von Zellstoff aufweist, **dadurch gekennzeichnet, dass** vor oder während des Dehnvorgangs die Materialbahn durch die Aufbringung von Wasser befeuchtet wird, und dass nach dem Dehnvorgang die Materialstruktur (22) durch das Verdampfen des Wassers fixiert wird, indem sie getrocknet wird, während die Spannung aufrechterhalten wird, so dass fixierende Papierverbindungen in der Materialstruktur (22) ausgebildet werden.

10. Verfahren nach Anspruch 6 oder 7, mit der Verwendung eines Bindemittels, **dadurch gekennzeichnet, dass** das Bindemittel an die Materialbahn vor, während oder nach dem Dehnvorgang aufgebracht wird, und dass nach dem Dehnvorgang die Materialstruktur (22) durch Querverbinden des Bindemittels fixiert wird.

**11.** Absorbierendes Wegwerfprodukt, wobei das Wegwerfprodukt (34) eine Längsrichtung und eine Querrichtung aufweist, und einen Vorderteil (35), einen Hinterteil (36) mit einem Schrittteil (37) zwischen diesen aufweist, wobei das Wegwerfprodukt ein Vorder flächenmaterial (38) aufweist, das dafür vorgesehen ist, sich während der Verwendung in Berührung mit der Haut des Trägers zu befinden, und ein Hinterflächenmaterial (40) aufweist, das dafür vorgesehen ist, sich während der Verwendung in Berührung mit der Kleidung des Trägers zu befinden, wobei das Wegwerfprodukt (34) ferner einen Absorptionskern (41) aufweist, der zwischen dem Vorderflächenmaterial (38) und dem Hinterflächenmaterial (40) liegt, und bei dem das Vorderflächenmaterial (38) wenigstens in Verbindung mit dem Schrittteil (37) ein flüssigkeitsdurchlässiges Oberflächenmaterial (39) aufweist, wobei das Oberflächenmaterial (39) im Mikromaßstab eine Materialstruktur mit zahlreichen Poren mit Porenquerschnittsabmessungen in dem Bereich 1 µm bis 150 µm aufweist, wobei jede der zahlreichen Poren in der Ebene des Oberflächenmaterials (39) eine erste Porenquerschnittsabmessung, eine zweite Porenquerschnittsabmessung, eine Porenquerschnittsfläche und einen Porenumfang aufweist, wobei die zweite Porenquerschnittsabmessung im wesentlichen unter einem rechen Winkel zu der ersten Porenquerschnittsabmessung verläuft, **dadurch gekennzeichnet, dass** das flüssigkeitsdurchlässige Oberflächenmaterial (21) in einem gedehnten und dauerhaft verformten Zustand fixiert ist und eine erste Porenquerschnittsabmessung (2a) aufweist, die erheblich größer ist als die zweite Porenquerschnittsabmessung (2b), um einen geringeren Druckabfall zu erhalten, wenn Flüssigkeit hindurch fließt.

**12.** Absorbierendes Wegwerfprodukt nach Anspruch 11, **dadurch gekennzeichnet, dass** verglichen mit dem Zustand vor dem Dehnvorgang die Porenquerschnittsfläche verringert ist, und der Porenumfang im wesentlichen unverändert bleibt.

**13.** Absorbierendes Wegwerfprodukt nach Anspruch 11 oder 12, wobei der Druckabfall über das flüssigkeitsdurchlässige Oberflächenmaterial (39) gemäß der Formel acht mal der Porenquerschnittsfläche geteilt durch den Porenumfang eingeschätzt wird, **dadurch gekennzeichnet, dass** die erste Porenquerschnittsabmessung in dem flüssigkeitsdurchlässigen Oberflächenmaterial (39) des absorbierenden Wegwerfprodukts (34) wenigstens 1,8 mal größer ist als die zweite Porenquerschnittsabmessung, und der Porenumfang höchstens 110% des Porenumfangs vor dem Dehnvorgang beträgt, dem das flüssigkeitsdurchlässige Oberflächenmaterial (39) unterworfen wurde, bevor es an das absorbierende Wegwerfprodukt (34) angebracht wurde.

**14.** Absorbierendes Wegwerfprodukt nach einem der Ansprüche 11 - 13, wobei das flüssigkeitsdurchlässige Oberflächenmaterial (39), bevor es an das absorbierende Wegwerfprodukt (34) angebracht wird, eine Längsrichtung und eine Querrichtung aufweist, und thermoplastisches Material mit einer Aufweichtemperatur aufweist, **dadurch gekennzeichnet, dass**, bevor es an das absorbierende Wegwerfprodukt (34) angebracht wird, das flüssigkeitsdurchlässige Oberflächenmaterial (39) auf eine Temperatur höher als die Aufweichtemperatur erwärmt wurde, unter Spannung um wenigstens 20% in entweder der Längsrichtung oder der Querrichtung gedehnt wurde, und dass das flüssigkeitsdurchlässige Material (39) ferner auf eine Temperatur niedriger als die Aufweichtemperatur gekühlt wurde, während die Spannung gehalten wird.

**15.** Absorbierendes Wegwerfprodukt nach einem der Ansprüche 11 - 13, **dadurch gekennzeichnet, dass**, bevor das flüssigkeitsdurchlässige Oberflächenmaterial (39) an das absorbierende Wegwerfprodukt (34) angebracht wird, die Materialstruktur des flüssigkeitsdurchlässigen Oberflächenmaterials (39) in einem gedehnten Zustand fixiert wurde, indem es mit Wasser befeuchtet wird und getrocknet wird, oder durch die Aufbringung eines Bindemittels und Setzen des Bindemittels.

## Revendications

**1.** Matériau de surface perméable aux liquides pour produits absorbants jetables, ledit matériau de surface (21) comprenant une structure de matériau (22) à petite échelle avec de nombreux pores (23, 23', 23"), avec des dimensions de section transversale de pore comprises dans la plage de 1 µm à 150 µm, chacun (23) desdits pores ayant, dans le plan du matériau de surface (21), une première dimension de section transversale de pore (2a), une deuxième dimension de section transversale de pore (2b), une aire de section transversale de pore et une circonférence de pore, ladite deuxième dimension de section transversale de pore (2b) étant essentiellement à angle droit avec ladite première dimension de section transversale de pore (2a), **caractérisé en ce que** le matériau de surface (21) perméable aux liquides étiré dans la direction longitudinale ou transversale et de ce fait, déformé de façon permanente, et fixé dans cet état, a une première dimension de section transversale de pore (2a) qui est considérablement plus grande que la deuxième dimension de section transversale de pore (2b), permettant d'obtenir de ce fait une faible chute de pression quand du liquide le traverse.

**2.** Matériau de surface perméable aux liquides selon la revendication 1, **caractérisé en ce que**, suite au processus d'étirement, l'aire de section transversale de pore a été réduite et la circonférence de pore est restée essentiellement inchangée par rapport à avant ledit processus d'étirement.

**3.** Matériau de surface perméable aux liquides selon la revendication 1 ou 2, dans lequel la chute de pression est estimée selon la formule huit fois l'aire de section transversale de pore divisé par la circonférence de pore, **caractérisé en ce que** la première dimension de section transversale de pore (2a) lors de l'application du matériau de surface (21) perméable aux liquides dans un produit absorbant jetable est au moins 1,8 fois plus grande que la deuxième dimension de section transversale de pore (2b), et **en ce que** la circonférence de pore vaut au plus 110 % de la circonférence de pore avant le processus d'étirement.

**4.** Matériau de surface perméable aux liquides selon l'une quelconque des revendications 1 à 3, ledit matériau de surface (21) ayant une direction longitudinale (X) et une direction transversale (Y) et comprenant un matériau thermoplastique pourvu d'une température de ramollissement, **caractérisé en ce que** pendant ledit processus d'étirement, le matériau de surface (21) perméable aux liquides a été chauffé à une température supérieure à ladite température de ramollissement et a été étiré sous tension d'au moins 20 % soit dans ladite direction longitudinale (X) soit dans ladite direction transversale (Y), et **en ce que** en connexion avec ledit processus d'étirement le matériau (21) perméable aux liquides a été refroidi à une température inférieure à ladite température de ramollissement tout en retenant ladite tension.

**5.** Matériau de surface perméable aux liquides selon l'une quelconque des revendications 1 à 3, **caractérisé en ce que** en connexion avec ledit processus d'étirement la structure de matériau (22) du matériau de surface (21) perméable aux liquides a été fixée dans un état étiré par humectage à l'eau et séchage, ou par l'application d'un agent liant et durcissement de l'agent liant.

**6.** Procédé de fabrication d'un matériau de surface perméable aux liquides pour produits absorbants jetables, ledit procédé comprenant le fait de fournir une bande de matériau (11) perméable aux liquides comprenant une structure de matériau (12) à petite échelle avec de nombreux pores (13, 13', 13"), chacun (13) desdits pores ayant une première dimension de section transversale de pore (d), une deuxième dimension de section transversale de pore (d'), une aire de section transversale de pore et une circonférence de pore, ladite deuxième dimension de section transversale de pore (d') étant essentiellement à angle droit avec ladite première dimension de section transversale de pore (d) et la bande de matériau (11) a une direction longitudinale et une direction transversale, **caractérisé en ce que** la bande de matériau (11) est déformée de façon permanente en la soumettant à un processus d'étirement sous tension d'au moins 20 % soit dans ladite direction longitudinale soit dans ladite direction transversale de sorte que la première dimension de section transversale de pore (2a) devient considérablement plus grande que la deuxième dimension de section transversale de pore (2b), et **en ce que**, ensuite, la structure de matériau (2b) de la bande de matériau obtenue est fixée afin de fournir un matériau de surface (21) perméable aux liquides ayant une chute de pression réduite quand du liquide le traverse.

**7.** Procédé selon la revendication 6, **caractérisé en ce que** l'aire de section transversale de pore est réduite et la circonférence de pore reste essentiellement inchangée après lesdits processus d'étirement et de fixation.

**8.** Procédé selon la revendication 6 ou 7, dans lequel la bande de matériau (11) comprend un matériau thermoplastique ayant une température de ramollissement, **caractérisé en ce que** avant ou pendant ledit processus d'étirement, la bande de matériau est chauffée à une température supérieure à ladite température de ramollissement, et **en ce que** après le processus d'étirement, la structure de matériau (22) est fixée par refroidissement à une température inférieure à ladite température de ramollissement tout en maintenant ladite tension.

**9.** Procédé selon la revendication 6 ou 7, dans lequel la bande de matériau (11) comprend des fibres à base de cellulose, **caractérisé en ce que** avant ou pendant ledit processus d'étirement, la bande de matériau est humidifiée par l'application d'eau, et **en ce que** après le processus d'étirement la structure de matériau (22) est fixée par l'évaporation de ladite eau par séchage tout en maintenant ladite tension de telle sorte que des liaisons de papier de fixation sont formées dans ladite structure de matériau (22).

**10.** Procédé selon la revendication 6 ou 7, comprenant l'utilisation d'un agent liant, **caractérisé en ce que** ledit agent liant est appliqué à la bande de matériau avant, pensant ou après ledit processus d'étirement, et **en ce que** après le processus d'étirement, la structure de matériau (22) est fixée en faisant réticuler ledit agent liant.

**11.** Produit absorbant jetable, ledit produit jetable (34) ayant une direction longitudinale et une direction transversale et comprenant une partie avant (35), une partie arrière (36) avec une partie d'entrejambe (37) entre elles, le produit jetable comprenant également un matériau de face avant (38) destiné à être au contact de la peau de l'utilisateur lors de l'utilisation, et un matériau de face arrière (40) destiné à être au contact des vêtements de l'utilisateur lors de l'utilisation, le produit jetable (34) comprenant également un noyau absorbant (41) se trouvant entre ledit matériau de face avant (38) et ledit matériau de face arrière (40) et dans lequel le matériau de face avant (38) comprend, au moins en connexion avec la partie d'entrejambe (37), un matériau de surface (39) perméable aux liquides, ledit matériau de surface (39) comprenant une structure de matériau à petite échelle avec de nombreux pores, avec des dimensions de section transversale de pore comprises dans la plage de 1 µm à 150 µm, chacun desdits pores ayant dans le plan du matériau de surface (39) une première dimension de section transversale de pore, une deuxième dimension de section transversale de pore, une aire de section transversale de pore et une circonférence de pore, ladite deuxième dimension de section transversale de pore étant essentiellement à angle droit avec ladite première dimension de section transversale de pore, **caractérisé en ce que** le matériau de surface (21) perméable aux liquides est fixé dans un état étiré et déformé de façon permanente, et a une première dimension de section transversale de pore (2a) qui est considérablement plus grande que la deuxième dimension de section transversale de pore (2b) afin d'obtenir une faible chute de pression quand du liquide le traverse.

**12.** Produit absorbant jetable selon la revendication 11, **caractérisé en ce que** l'aire de section transversale de pore a été réduite et la circonférence de pore reste essentiellement inchangée, par rapport à avant ledit processus d'étirement.

**13.** Produit absorbant jetable selon la revendication 11 ou 12, dans lequel la chute de pression dans le matériau de surface (39) perméable aux liquides est estimée selon la formule huit fois l'aire de section transversale de pore divisé par la circonférence de pore, **caractérisé en ce que** la première dimension de section transversale de pore du matériau de surface (39) perméable aux liquides du produit absorbant jetable (34) est au moins 1,8 fois plus grande que la deuxième dimension de section transversale de pore, et la circonférence de pore vaut au plus 110 % de la circonférence de pore avant le processus d'étirement auquel le matériau de surface (39) perméable aux liquides a été soumis avant d'être appliqué sur le produit absorbant jetable (34).

**14.** Produit absorbant jetable selon l'une quelconque des revendications 11 à 13, dans lequel avant d'être appliqué sur le produit absorbant jetable (34), le matériau de surface (39) perméable aux liquides a une direction longitudinale et une direction transversale et comprend un matériau thermoplastique pourvu d'une température de ramollissement, **caractérisé en ce que** avant d'être appliqué sur le produit absorbant jetable (34), le matériau de surface (39) perméable aux liquides a été chauffé à une température supérieure à ladite température de ramollissement et a été étiré sous tension d'au moins 20 % soit dans ladite direction longitudinale soit dans ladite direction transversale, et **en ce que** le matériau (39) perméable aux liquides a aussi été refroidi à une température inférieure à ladite température de ramollissement tout en retenant ladite tension.

**15.** Produit absorbant jetable selon l'une quelconque des revendications 11 à 13, **caractérisé en ce que** avant que le matériau de surface (39) perméable aux liquides soit appliqué sur le produit absorbant jetable (34) la structure de matériau du matériau de surface (39) perméable aux liquides a été fixée dans un état étiré par humectage à l'eau et séchage, ou par l'application d'un agent liant et durcissement de l'agent liant.

_FIG.1_

_FIG.2_

FIG.3